# EUROPEAN PATENT SPECIFICATION

(11) **EP 0 236 476 B1**
(45) Date of publication and mention of the grant of the patent: **01.12.1993**
(21) Application number: 86906050.9
(22) Date of filing: 04.09.1986
(51) Int. Cl.: A61K 31/00, A61K 31/02

(54) **OXYGENATED PERFLUORINATED PERFUSION OF THE OCULAR GLOBE**
INFUSION SAUERSTOFFREICHER PERFLUORIERTER FLÜSSIGKEIT IM AUGAPFEL
PERFUSION PERFLUOREE OXYGENEE DU GLOBE OCULAIRE

(30) Priority: 10.09.1985 GB 8522374
(43) Date of publication of application: 16.09.1987
(73) Proprietor: THOMAS JEFFERSON UNIVERSITY, Philadelphia Pennsylvania 19107 (US)
(72) Inventor: OSTERHOLM, Jewell, L., Radnor, PA 19087 (US)
(74) Representative: Baillie, Iain Cameron
(86) International application number: US8601829
(87) International publication number: WO8701271

(56) References cited:
- EP-A- 121 277
- EP-A- 0 089 232
- US-A- 4 067 971
- US-A- 4 106 992
- US-A- 4 340 037
- US-A- 4 445 886
- US-A- 4 450 841
- US-A- 4 496 342
- US-A- 4 550 022
- Klinisches Monatsblatt Augenheilkunde, vol. 186, June 1985, pp. 485-487, Enke Verlag, Stuttgart, DE C.J. Pournaras et al.
- Ocular Manifestations of Diabetes, vol. 24, no. 4, Winter 1984, IOC, Little, Brown and Company, Boston, US, ed. Peter Reed Pavan; S.M. Buzney et al., pp. 1-12
- Am. J. of Ophthalmology, vol. 96, no. 4, Oct. 1983, pp. 510-516; H. Lincoff et al.
- Ophthalmology, vol. 90, no. 5, 1983, pp. 484-487, Am. Acad. of Ophthalmology, US; E.M. Kohner et al.

## Description

The present invention relates to pharmaceuticals for treating ischemic retinopathy, particularly that caused by retinal infarction.

Ischemic retinopathy is a major cause of blindness. Retinal ischemia may result from a number of different causes and may be associated with other diseases and conditions, such as diabetes, atherosclerosis, etc. For example, retinal ischemia caused by central retinal vein occlusion (CRVO). CRVO may result from a number of different underlying conditions.

A number of treatments have been suggested for retinal ischemia. For example, to manage outflow obstruction, it has been suggested to administer fibrinolytic agents and anticoagulants, to conduct hemodilution and plasma exchange, to administer steroids, or to conduct photocoagulation. See Kohner et al, "The Management of Central Retinal Vein Occlusion", Ophthalmology, 90(5): 484-487 (1983). Unfortunately, the aforementioned treatments have not been found very effective. See also Hansen et al, "A Randomised Prospective Study on Treatment of Central Retinal Vein Occlusion by Isovolaemic Haemodilution and Photocoagulation", British Journal of Ophthalmology, 69:108-116 (1985).

The prognosis for diabetic retinal angiopathy is not very encouraging. See Buzney et al, "Pathogenesis of Diabetic Retinal Angiopathy: Proposed Mechanisms and Current Research", International Ophthalmology Clinics, 24(4):1-11 (Winter, 1984). Buzney et al recognize that a need exists to develop new methods of treating diabetic retinal angiopathy, suggesting that research efforts will "someday produce chemotherapeutic methods that will exceed the scope of laser or vitreoretinal surgery". Buzney et al hypothesize that the systemic use of such chemotherapeutic agents may be supplemented "by injection into the vitreous chamber or even the use of a vitreous substitute that fully tamponades the retina, inactivating or selecting inhibiting the diffusion of vasoactive substances". See Buzney et al at page 9.

While some of the aetiologies of retinal vein occlusion are well understood, others are only partially so, and in still others the cause remains obscure. Systemic hypertension is recognized as a cause of certain occlusions, as is the inflammation of the vein wall (periphlebitis) and glaucoma. As discussed in Kanski et al, "Disorders of the Vitreous, Retina and Choroid", Ophthalomology I, pp. 115-121, Butterworths International Medical Reviews, London, (1983), the conventional treatments for retinal vein occlusion are systemic drug administration and photocoagulation, neither of which are consistently effective.

Although generally unrelated to the management of central retinal vien occlusion or other retinal ischemias, it is known to introduce a variety of liquids or gases into the ocular globe for a variety of reasons. For example, in Chandler et al, "A Refined Experimental Model for Proliferative Vitreoretinopathy", Graefe's Arch. Clin. Exp. Ophthalmol, 224:86-91 (1986), experiments are described wherein the intact vitreous region is injected with large numbers of tissue cultured fibroblasts. However, such injections were not found to induce the disease as it is found in humans. In Lincoff et al, "Use of an Intraocular Gas Tamponade to Find Retinal Breaks", American Journal of Ophthalmology, 96:510-516 (1983), the subretinal fluid was drained and the volume replaced by a perfluorocarbon gas calculated to fill the eye below the probable level of the retinal break. The bubble closed the break and maintained reattachment until the gas was absorbed.

In Kreissig et al, "The Treatment of Difficult Retinal Detachments with an Expanding Gas Bubble Without Vitrectomy", Graefe's Arch. Clin. Exp. Ophthalmology 224:51-54 (1986). A prospective study is reported using perfluorocarbon gases (CF₄, C₂F₆, C₃F₈) without prior mechanical vitrectomy.

In Haut et al, "Some of the Most Important Properties of Silicone Oil to Explain its Action", Ophthalmologica, Basel 191:150-153 (1985), the action of silicone oil used to close tears and reattach the retina is disclosed. The action of such silicone oil is described as being a result of its density and surface tension causing the bubble to press upon the upper part of the eye, i.e., most of the time at twelve o'clock so there is constant support upwards which closes the tears and reattaches the retina.

Notwithstanding what is known in this area, there is a long felt need for effective pharmaceuticals to for treating ischemic retinopathy.

The present invention provides a novel use of a physiologically acceptable oxygenated fluid to prepare a medicament for treating ischemic retinopathy and/or retinal ischemia. For use of the medicament, the treatment comprises the steps of removing all or at least a portion of the vitreous body to create an intraocular perfusion space, and establishing a perfusion of physiologically compatible oxygenated nutrient liquid through said perfusion space to support the metabolic needs of the retina until natural healing occurs.

Preferably, the step of removing a portion of the vitreous body comprises creating an incision at the limbus of the eye adjacent to the irido-corneal angle, and then using an ultrasonic desecrator. The amount of vitreous body which is actually removed will depend to some extent upon the effect of such removal upon the condition of the underlying retina, however, in any event, a sufficient portion, if not all, of the vitreous body will be removed such that the subsequent establishment of a perfusion through the chamber thus created will be sufficient to expose the ischemic region of the retina to effective amounts of perfused oxygenated liquid.

A circulatory perfusion of physiologically compatible oxygenated nutrient liquid is established by inserting input and output cannulae through the irido-corneal region of the eye, suturing them in place. Using an oxygenated perfluorocarbon emulsion of the type disclosed in the aforementioned Osterholm U.S. patents (which have been incorporated by reference herein), a perfusion rate of between 0.25 to 10 (cm³) ml per minute is established at a perfusion pressure which is no greater than the normal vitreous pressure of the eye. The subject perfusion is established through said cannulae, which are preferably 18 to 25 guage cannulae, the inner termini of which are suitably positioned within the intraocular perfusion space created by the removal of the vitreous body to thereby cause oxygenated perfluorocarbon emulsion to wash across the ischemic region to be treated. The oxygenated liquid should then be perfused for 3-5 days at a rate which is sufficient to support the metabolic needs of the retina until normal healing occurs.

In order to determine the progress of such healing and/or the sufficiency of the subject treatment to maintain the metabolic needs of the retina, perfusion with oxygenated fluorocarbon emulsion, which is a milky white substance, should be periodically interrupted in favor of a clear physiologic saline solution which will allow visual observation of the retina and, depending upon the patient's condition, vision.

At the conclusion of treatment, any residual perfluorocarbon emulsion is washed from the intraocular perfusion space and is replaced with a conventional synthetic vitreous solution or gel to replace the vitreous body, the cannulae are removed, and the ocular globe is resealed. Since a relatively small surface area of tissue is exposed to the perfluorocarbon, and since the perfluorocarbon emulsion is formulated to be physiologically compatible, systemic side effects should not be observed.

A further use of the composition is in preparing a diagnostic tool for use in diagnosing conditions within the eye.

The present invention also provides a novel method for diagnosing the condition of retinal tissue suspected of being ischemic. This method comprises the steps of removing at least a portion of vitreous body to create an intraocular perfusion space adjacent to the retina; establishing a circulation of a physiologically compatible liquid of known composition through said perfusion space by injecting said liquid into and withdrawing said liquid from said perfusion space; and analyzing said liquid withdrawn from said intraocular perfusion space to determine at least one characteristic of said withdrawn fluid which differs from said fluid as injected and which is representative of the condition of said retinal tissue. In accordance with this preferred diagnostic method, the withdrawn fluid is analyzed for any one of a variety of compositional characteristics, such as oxygen content, lactate acid concentration, carbon dioxide concentration, ammonia concentration, pH, etc. As a result, the attending physician may monitor the progress of the treatment for retinal ischemia and/or of underlying healing processes using objective physical or chemical means.

Accordingly, the present invention provides a pharmaceutical for treating retinal ischemia to prevent blindness. This and other objects of the present invention will become apparent from the following, more detailed description.

### Brief Description of the Drawings

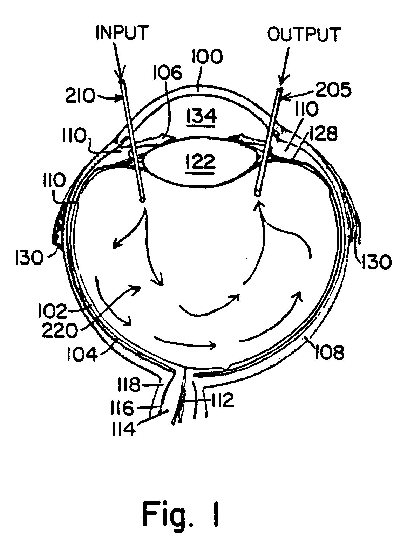

Figure 1 is a diagrammatic horizontal section of the eyeball illustrating the intraocular perfusion established by the method of the present invention.

### Detailed Description of the Preferred Embodiments

The present invention provides a pharmaceutical of treating retinal ischemia. As seen in Figure 1, the retina 102 is the internal coat of the eye. The retina 102 is disposed over the middle or vascular coat of the eye, which coat comprises the choroid 104, ciliary body 110 and iris 106. The eyeball further comprises an external or fibrous coat comprising the transparent cornea 100 and sclera 108. Behind the cornea is aqueous humor 134 which fills two chambers, an anterior one in front of the iris and a posterior one behind the iris. The lens 122 is located behind the aqueous humor and is suspended by suspensory ligaments which extend generally from the sides of the lens towards the ciliary process 128. The vitreous body, which is a jelly-like substance disposed within the vitreous membrane, is normally disposed behind the lens occupying the vitreous chamber. In the eye illustrated in Figure 1, however, the vitreous body has been removed to create in the vitreous chamber an intraocular perfusion space designated generally 220. Accordingly, the retina 102 which comprises an outer layer pigmented cells and an inner layer of optic cells (which layers are not shown in the drawing) are directly exposed to the intraocular perfusion space. The central artery and vein of the retina 112 supply the retina in the region of the optic nerve, which is surrounded by an internal sheath 114 and an intervaginal subarachnoid space 116. The nerve further comprises an external neural sheath 118. External muscles 130 attached to the sclera assist in rotating the eyeball.

In accordance with the preferred embodiment of the present invention, the entirety of the vitreous body is removed to create an intraocular perfusion space 220, adjacent to the portion of the retina to be treated. The vitreous body may be removed by creating an incision at the limbus of the eye adjacent to the irido-corneal angle, and using an ultrasonic desecrator with suction (such as the CUSA desecrator to remove the vitreous body. Following removal of the vitreous body, small catheters or cannulae 205 and 210 are inserted through the irido-corneal angle into the intraocular perfusion space 220 thus created. Those of ordinary skill in ocular surgery will recognize that the placement of these cannulae may differ somewhat from that shown in Figure 1. Each of catheters 205 and 210 are sutured water-tight in place. Once in place, an intraocular circulation should be established using a physiologic oxygenated liquid which is capable of providing for the metabolic needs of the affected retina.

The preferred oxygenated liquid of the present invention is that which is disclosed in the aforementioned Osterholm patents. See for example, the disclosure of U.S Patent 4,450,841 at column 15-20. The preferred physiologic oxygenated liquid of the present invention is a nutrient emulsion comprised of carefully formulated components including electrolytes (sodium, potassium, calcium, magnesium and chloride) and a non-aqueous oxygen transfer component (such as a perfluorobutyltetrahydrofuran which has been sold by the 3-M Corporation under the trademark "FC-80" or "RIEMAR's RM-101". The preferred non-aqueous oxygen transfer component of the preferred nutrient liquid should exhibit, when charged oxygen, vapor pressures in the range above 53,000 Pa (400 Torr), and preferably above 80,000 Pa (600 Torr). Such oxygen transfer components similarly should not exhibit high vapor pressures which would boil at body temperatures, nor have viscosities which are difficult if not impossible to emulsify. It is presently anticipated that other fluorocarbon compounds may be found suitable for use in performing the methods of the present invention, including such fluorocarbons as PFOB, those which can be emulsified at very small (less than 2 micrometer particle sizes) to be clear, and/or those containing 2 or more aromatic rings. While emulsions prepared from fluorocarbons such as perfluorobutyltetrahydrofuran are milky white in color, most desired would be to utilize a fluorocarbon emulsion which is clear, and which therefore would not interfere with vision or visual retinal observation during treatment. In addition to the aforementioned components, the subject emulsions should have an emulsification component, which may be any one of a number of known fluorocarbon emulsifiers, of which block polymer polyols, such as a pluronic, is representative. The osmolarity of the subject emulsion should be controlled within a range of 290-330 mOsM, with the slightly higher range of 220-230 being preferred to lessen swelling and reduce pressure within the eye. In addition to the aforementioned components, the subject emulsion preferably comprises glucose, amino acids, steroids, antibiotics, etc., as disclosed more fully in the aforementioned U.S. patents.

Once the vitreous body has been removed and the cannulae inserted, an intraocular perfusion may be established. During the intraocular perfusion, it may be desired to ensure the stability of the subject catheters by using an external appliance (Dutchman) to fix their positions with respect to the eye. Although two catheters are presently illustrated, it is also within the scope of the present invention to utilize a single double lumen catheter which has separate input and output locations to similarly create the desired intraocular perfusion across the retinal surface to be treated.

The oxygenated nutrient emulsion may be delivered at room temperature, that is at about 24°C., however higher or lower temperatures may be used to deliver the oxygenated nutrient emulsions as medical conditions warrant.

In accordance with the preferred method of the present invention, sufficient nutrient emulsion should be supplied to counteract oxygen and other metabolite deprivation of the effected retinal tissue. It is currently anticipated that perfusion rates as much as 10 ml/min may be established at normal vitreous pressures, however, flows of as little as 2.5 ml/min may be sufficient to treat certain affected regions of ischemic retinal tissue.

It is preferred to establish the circulation of physiologically compatible oxygenated liquid as quickly as possible upon the diagnosis of retinal infarction. The circulation may be maintained awaiting normal healing, or surgical procedures may be used to reestablish blood supply to the effected portion of the retina. In either event, once the retina is able to sustain itself without perfusion, the perfused liquid will be allowed to remain in the globe if transparent, or preferably may be replaced by physiologically compatible synthetic vitreous humor.

Accordingly, the method of the present invention takes advantage of the fact that the retina is of the same neural origin as the brain, and follow the same response to ischemia as does the brain. In these terms, while the retina has relatively poor tolerance to ischemia and undergoes infarction resulting in partial or complete visual loss, it is also susceptible to artificial maintenance through the use of physiologic oxygenated fluorocarbon emulsions which have been found suitable for maintaining the viability of neural tissue in other locations, such as the brain and spinal cord.

Anatomically, the optic globe has features analogous to the brain. The retina is adjacent to a body of fluid (vitreous humor), similar to the brain's relationship to the cerebrospinal fluid. The vitreous humor can be removed without lasting harm to vision as long as the shape of the globe is not permitted to undergo major distortion. Accordingly, the vitreous humor can be aspirated and replaced with a physiologic salt solution without perturbing visual function. This group of factors, i.e., anatomic proximity, replaceable fluid, and similarity of the retina to the brain all favor this approach to resuscitating retinal tissue which otherwise will be irretrievably lost.

It is also within the scope of the present invention to diagnosis the condition of the retina by analyzing the physical and chemical characteristics of the perfusate after it is withdrawn from output catheter 205. The fluid which is withdrawn from the intraocular perfusion space will not be of identical composition to the oxygenated nutrient emulsion which is injected through input catheter 210. By taking advantages of differences in the composition which are detected in the withdrawn fluid, which may be considered to have become a diagnostic fluid, the attending physician may easily monitor the physiologic condition of the neurologic retinal tissue which is being treated. This diagnostic fluid may also be monitored to assure that treatment is proceeding according to plan. Accordingly, fluid which is drawn from the intraocular perfusion space may be analyzed for characteristics including potassium and sodium ion concentration, lactic acid concentration, gamma aminobutyric acid (GABA) and other amino acid concentrations, oxygen concentration, carbon dioxide concentration, enzyme concentration, microorganism (bacterial) content, ammonia concentration, myelin fragments, cellular materials including organelles, proteins, fats, RNA, DNA, metabolites, metabolic products, pH and/or neurotransmitter content. This diagnostic method takes advantage of the fact that ischemic neurologic tissue produces higher concentrations of such materials as GABA, lactate ion, enzymes, and/or LDH (lactate dehydrogenase), ammonia, and other constituents which have been determined by analyzing cerebrospinal fluid of patients subjected by disease to anoxic conditions or neural tissue. In accordance with the method of the present invention, a continuous monitoring of the state of the neurologic tissue is possible since the circulation of oxygen in nutrient emulsion will produce a continuous flushing of the affected retinal tissue, and thus will result in diagnostic fluid component variations which are rapidly reflective of the physiologic state of the tissue being treated.

Accordingly, novel diagnostic methods are provided by the present invention for treating ischemic retinopathy, particular retinal infarctions, which otherwise may result in blindness.

## Claims

1. Use of a physiologically acceptable oxygenated fluid for preparing a medicament for treatment of retinal ischemia by introduction into intraocular region.

2. Use according to claim 1, of a physiologically acceptable oxygenated fluid consisting of an emulsion comprising electrolytes and an oxygen transfer agent, preferably a fluorocarbon, having a vapor pressure when charged with oxygen above 53,000 Pa (400 Torr) for preparing a medicament for treatment of retinal ischemia by introduction into intraocular region.

3. Use of a physiologically acceptable oxygenated fluid for preparing a diagnostic reagent for diagnosing conditions of the eye.

4. Use according to claim 3, of a physiologically acceptable oxygenated fluid consisting of an emulsion comprising electrolytes and an oxygen transfer agent for preparing a diagnostic agent for diagnosis conditions of the eye.

5. Method of collecting information permitting diagnosis comprising analyzing a perfusate that has been recovered after passage through an intra ocular perfusion space in the vitreous body of an eye, said analysis being carried out to determine one or more of: oxygen content, lactic acid concentration, carbon dioxide concentration, ammonia concentration and pH.

## Patentansprüche

1. Verwendung einer physiologisch zulässigen, mit Sauerstoff angereichertern Flüssigkeit zur Zubereitung eines Medikaments für die Behandlung von retinaler Ischämie durch Einführung in den intraokularen Bereich.

2. Verwendung einer physiologisch zulässigen, mit Sauerstoff angereicherten Flüssigkeit nach Anspruch 1, bestehend aus einer Emulsion, die Elektrolyte und ein Sauerstofftransportmittel umfaßt, vorzugsweise einen Fluorkohlenstoff, und nach Beladung mit Sauerstoff einen Dampfdruck von mehr als 53.000 Pa (400 Torr) aufweist, zur Zubereitung eines Medikaments für die Behandlung von retinaler Ischämie durch Einführung in den intraokularen Bereich.

3. Verwendung einer physiologisch zulässigen, mit Sauerstoff angereicherten Flüssigkeit zur Zubereitung eines diagnostischen Reagens zum Diagnostizieren von Erkrankungen des Auges.

4. Verwendung einer physiologisch zulässigen, mit Sauerstoff angereicherten Flüssigkeit nach Anspruch 3, bestehend aus einer Emulsion, die Elektrolyte und ein Sauerstofftransportmittel aufweist, zur Zubereitung eines diagnostischen Mittels für die Diagnose von Erkrankungen des Auges.

5. Methode zum Erfassen eine Diagnose ermöglichender Information, umfassend das Analysieren eines Perfusats, das nach Passage durch einen intraokularen Perfusionsraum im Glaskörper eines Auges wiedergewonnen wurde, wobei die Analyse ausgeführt wird, um einen oder mehrere der folgenden zu bestimmen: Sauerstoffgehalt, Milchsäurekonzentration, Konzentration an Kohlenstoffdioxid, Ammoniakkonzentration und pH.

## Revendications

1. Emploi d'un fluide oxygéné physiologiquement acceptable pour préparer un médicament destiné au traitement de l'ischémie rétinienne, par introduction dans la région intra-oculaire.

2. Emploi selon la revendication 1, d'un fluide oxygéné physiologiquement acceptable consistant en une émulsion comprenant des électrolytes et un agent de transport de l'oxygène, de préférence un carbure fluoré ayant lorsqu'il est chargé en oxygène une pression de vapeur supérieure à 53 000 Pa (400 torrs), pour préparer un médicament destiné au traitement de l'ischémie rétinienne par introduction dans la région intra-oculaire.

3. Emploi d'un fluide oxygéné physiologiquement acceptable pour préparer un réactif de diagnostic destiné au diagnostic des pathologies oculaires.

4. Emploi selon la revendication 3 d'un fluide oxygéné physiologiquement acceptable consistant en une émulsion comprenant des électrolytes et un agent de transport de l'oxygène pour préparer un agent de diagnostic destiné au diagnostic des pathologies oculaires.

5. Procédé de recueil d'informations permettant le diagnostic, comprenant l'analyse d'un perfusat récupéré après son passage par un espace de perfusion intra-oculaire dans le corps vitré d'un oeil, ladite analyse étant effectuée pour déterminer un ou plusieurs des éléments suivants : teneur en oxygène, concentration en acide lactique, concentration en dioxyde de carbone, concentration en ammoniac et pH.
